# EUROPEAN PATENT SPECIFICATION

(11) **EP 2 433 640 B1**
(45) Date of publication and mention of the grant of the patent: **15.01.2020**
(21) Application number: 10306032.3
(22) Date of filing: 24.09.2010
(51) Int. Cl.: A61K 38/44, A61K 31/07, A23L 5/42, A23L 29/00, A23L 33/105, A23L 33/15, A23L 33/17

(54) **Composition comprising SOD, lutein and zeaxanthin**
Zusammensetzung mit SOD, Lutein und Zeaxanthin
Composition comprenant de la superoxyde dismutase, de la lutéine et de la zéaxanthine

(43) Date of publication of application: 28.03.2012
(73) Proprietor: OmniVision GmbH, 82178 Puchheim (DE)
(72) Inventor: Hadj-Slimane, Réda, 75015 Paris (FR); Hadj-Slimane,Tewfik, 48000 Relizane (DZ); Lepelletier, Yves, 91200 Athis-Mons (FR)
(74) Representative: Blodig, Wolfgang

(56) References cited:
- WO-A1-96/21462
- WO-A2-03/082081
- US-A1- 2005 032 768
- US-A1- 2007 265 351
- US-A1- 2010 159 029
- LEE SUNGMUN ET AL: "Polyketal microparticles: A new delivery vehicle for superoxide dismutase", BIOCONJUGATE CHEMISTRY, vol. 18, no. 1, January 2007 (2007-01), pages 4-7, XP002630444, ISSN: 1043-1802

## Description

### FIELD OF INVENTION

The present invention relates to a composition comprising superoxide dismutase (SOD) in association with lutein and at least one stereoisomer of zeaxanthin. Said composition is a nutraceutical composition or a pharmaceutical composition, and promotes or maintains visual health particularly in AMD, through the inhibition of oxidative damages.

### BACKGROUND OF THE INVENTION

In developed countries, the most frequent causes of visual incapacities or blindness are diseases, such as for example age-related macular degeneration (AMD), diabetic retinopathies (DR), cataract and retinitis pigmentosa (RP). These different ocular pathologies present the common characteristic to be associated with oxidative stress.

Oxidative stress corresponds to an excess of oxidant elements as compared to antioxidant products, resulting in structural or formal damages, such as cellular damages. Oxidative stress is linked to an excessive production of reactive oxygen species (ROS) coupled to a deficiency in anti-radical defenses. In particular, oxidative stress leads to the accumulation of toxic superoxide ion (O₂⁻). In retina, the oxidation of lipids linked to amino acids leads to the accumulation of lipofuscine, a fluorescent pigment. This pigment induces the production of ROS leading to cell death by apoptosis (Wihlmark et al., Free radic. Biol. Med. 1997; 22:1229-34). Moreover, with ageing, antioxidant defenses decrease, in particular in the eye.

With global ageing of the population, the occurrence of the cited diseases increases. The provoked symptoms are highly invalidating for patients. There is, thus, a real need to develop strategies to counterbalance oxidative stress in the eye, in order to prevent and/or treat said diseases.

In human, antioxidant protection involves two major types of elements: enzymes or non-enzymatic products.

Three major antioxidant enzymes are present in human cells: glutathione reductase and peroxydase (GPX), superoxide dismutase (SOD) and catalase (CAT). The combination of the action of these three enzymes leads to the transformation of highly toxic superoxide ion to hydrogen peroxide (H₂O₂) and finally to water, as shown by the chemical reactions below.

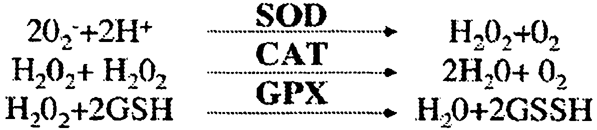

The use of SOD, for example from *Cucumis melo* LC., to treat diseases associated with oxidative stress is well known in the prior art. US2003/0228299 describes the use of pharmaceutical compositions comprising SOD for topical administration, in order to prevent and/or treat superficial eye disorders. However, oral administration of SOD has longer been limited, due to the degradation of the enzyme through the digestive tract (Zidenberg-Cherr et al., The American Journal of Clinical Nutrition, 1983). US 6,045,809 and Dugas (Free Radic Biol Med, 2002), both describe the use of gliadin biopolymers to protect SOD from digestive degradation. A form of SOD protected by gliadin is available as GliSODin® (Isocell). The catalytic activity of SOD protected by gliadin is preserved during oral administration (Vouldoukis et al., Journal of Ethnopharmacology, 2004), allowing its administration through an oral way.

In parallel to enzymatic protection of cells against oxidative stress, some non-enzymatic products exist. For example, vitamins (A, E or C), zinc, selenium, anthocyanins or macular pigments, such as, for example, lutein and zeaxanthin are usually used for their antioxidant properties. Lutein and zeaxanthin are macular pigments representing 99% of total pigments of the macula, which belong to the family of carotenoids, and specifically to the family of xanthophylls. Both pigments may exhibit on one hand an indirect antioxidant effect through their capacity to absorb blue light particularly aggressive for photoreceptor and on the other hand direct antioxidant properties. US2005/0032914, US2010/0159029 and WO2009/129859 describe composition for oral administration, comprising lutein and zeaxanthin in combination with other non-enzymatic antioxidant products, for enhancing visual performance, inhibiting macular degeneration or promoting eye health. Moreover, lutein is defined by the Afssa (Agence Française de Sécurité Sanitaire des Aliments) as "an agent which contributes to protect retina and lens against oxidation" (Saisine n°2003-SA-0205, 2004) and scientific studies have shown a relationship between the dietary intake of lutein/zeaxanthin and the likelihood of having AMD (SanGiovanni et al., AREDS report n°22, Arch Ophthalmol, 2007).

CN 101243875 describes a health preparation, comprising lutein and beta-carotene to enhance the activity of SOD. The Applicant discloses the fact that lutein alone is less efficient to enhance the activity of SOD than in combination with beta-caroten.

Surprisingly, and contrary to the teaching of the prior art, especially CN 101243875, the Applicant identified a synergic effect of oral administration of SOD protected by gliadin, lutein and at least one isomer of zeaxanthin on global health, and on the protection against oxidative stress, without the addition of beta-carotene. Moreover, the Applicant carried out experiments showing that this synergy is enhanced when components are administered to a subject non-simultaneously.

### SUMMARY

This invention thus relates to a composition comprising superoxide dismutase (SOD) in association with lutein and at least one isomer of zeaxanthin, preferably of one stereoisomer of zeaxanthin.

Advantageously, superoxide dismutase (SOD) is of natural origin, and the composition comprises a vegetal extract including SOD, preferably an extract of *Cucumis melo* LC, preferably the amount of vegetal extract including superoxide dismutase ranges from 10 to 2000 mg, preferably from 50 to 1000 mg, more preferably 100-500 mg. According to a preferred embodiment, the enzymatic activity of SOD is of at least 1.1 UI/mg. Advantageously, SOD is micro-encapsulated with a biopolymer, preferably a prolamine or PCADK, and optionally the micro-encapsulated SOD is further coated with a polymer, preferably chitosan or hyaluronic acid.

In an embodiment, the at least one zeaxanthin isomer is selected from the group consisting of zeaxanthin and mesozeaxanthine. In a further embodiment, the amount of lutein ranges from 0.5 to 25 mg, and the amount of at least one zeaxanthin isomer ranges from 0.1-5 mg. Advantageously, lutein and at least one zeaxanthin isomer are present in the composition in a ratio lutein:zeaxanthin isomer of about 5:1.

In a preferred embodiment, the association is an intimate mixture of superoxide dismutase lutein and at least one zeaxanthin isomer.

This invention also relates to a kit of parts comprising a composition as described here above, wherein the kit comprises a first part comprising superoxide dismutase, and a second part comprising lutein and at least one zeaxanthin isomer. Advantageously, one of the first and second parts is formulated for sustained release thereof. In an embodiment, the first and second parts are administered to an individual simultaneously or sequentially, and when administered sequentially, the first part comprising the enzyme is administered 12 hours before or after the second part comprising lutein and at least one zeaxanthin isomer.

This invention also relates to a functional food, a nutraceutical composition or a food or dietary supplement comprising a composition or a kit of parts as described here above. Advantageously, the functional food, the nutraceutical composition or the food or dietary supplement comprises 50-200 mg, preferably about 100 mg enzyme, 2.5-7.5 mg, preferably about 5 mg lutein and 0.5-1.5 mg, preferably about 1 mg zeaxanthin. It may be in a form suitable for being orally administered.

This invention also relates to a pharmaceutical composition comprising a composition or a kit of parts as described here above in association with at least one pharmaceutically acceptable excipient. This invention also relates to a medicament comprising a composition or a kit of parts as described here above. The pharmaceutical composition or the medicament according to the invention may comprise 100-1000 mg superoxide dismutase, 1-25 mg lutein and 0.5-5 mg zeaxanthin. They may be in a form suitable for being orally, topically, intraocularly or systemically administered.

This invention also relates to a veterinarian product, comprising a composition or a kit of parts as described here above.

This invention also relates to a composition for use in treating, preventing or stabilizing a disease, condition or disorder of the eye associated to oxidative stress, comprising administering to a subject in need thereof a pharmaceutical composition or a medicament as described here above. The subject may be affected by a disease associated with oxidative stress, especially Age-related Macular Degeneration (AMD), diabetic retinopathies, cataract or retinitis pigmentosa. According to an embodiment, the subject is an animal, preferably a mammal, such as, for example, cat, dog, hamster, rabbit, mouse, gerbil, rat, Guinea pig, human, more preferably the subject is a human.

### DEFINITIONS

In the present invention, the following terms have the following meanings:
- **"Antioxidant"** refers to an enzymatic or non enzymatic agent that diminishes or avoids the oxidation of other substances. An antioxidant may protect cells against the effect of reactive oxygen species.
- **"Oxidative stress":** imbalance between the production of reactive oxygen species on one hand and on the other hand the detoxification of the reactive intermediates and/or the repair of the resulting damages.
- "**Oral administration**": the administration of a product in the mouth cavity followed by the swallowing of the product. The substance reaches the systemic circulation through a digestive absorption.
- "**Topical administration**": defines the delivery of a product by application to the skin or mucous membrane, preferably a topical administration refers to the application of a product on the surface of an eye.
- "**Intraocular administration**": refers to the injection of a product directly in the eyeball, insight the anterior chamber, the posterior chamber or the vitreous chamber, preferably insight the vitreous chamber.
- **"Systemic injection":** refers to the injection of a product in the animal body, wherein the product reaches the systemic circulation.
- **"Functional food":** any modified food or food ingredient that may provide a benefit or provide protection against physiological disorder or discomfort; beyond the traditional nutrients it contains.
- "**Nutraceutical product**": a product isolated or purified from comestibles. A nutraceutical is demonstrated to have a physiological benefit or provide protection against physiological disorder or discomfort.
- "**Food or dietary supplement**": a product that contains a vitamin, mineral, herb or other botanical, amino acid, concentrate, metabolite, constituent, extract, or combinations of these ingredients.
- **"Polymer":** large molecule formed by the repetition of a structural unit.
- "**Biopolymer**": defines a polymer produced by living organism, such as, for example, DNA, cellulose, starch, gliadin, chitosan, hyaluronic acid, cyclodextrine, polysome, etc...
- **"Sustained release":** describes the slow release kinetic of a compound, over an extended period of time, preferably 12 hours or more.
- **"About"** preceding a figure means plus or less 10% of the value of said figure
- "**Association**": refers to a combination of products, or to at least two products which are to be administered or ingested by a subject in a simultaneous or sequential manner, in order to have a combined effect.
- "**Intimate mixture**": refers to the close union or combination of elements.
- "UI": 1 UI corresponds to the transformation of one micromole of substrate (ROS) per minute at 25°C.
- "**maintaining Visual health**" means alleviating discomforts which may result from oxidative stress and that may cause on a long run visual damages such as for example AMD. An example of oxidative stress resulting in visual discomforts is the UV exposure inducing phototoxicity. Oxidative stress is largely found in AMD, DR or RP.
- "**Subject**" or "**individual**": refers to an animal, preferably a mammal, more preferably a human, receiving the composition of the invention.

### DETAILED DESCRIPTION

The invention relates to a composition comprising or consisting of superoxide dismutase (SOD) in association with lutein and at least one stereoisomer of zeaxanthin.

In a first embodiment of the invention, SOD is synthetic SOD. Advantageously, synthetic SOD is recombinant SOD, provided from vegetal or animal, such as, for example human, sequences cloned and produced in a prokaryote system, such as, for example, a bacterial system; in an eukaryote system, such as, for example in a fungus, such as, for example, a yeast, or in mammalian cell such as, for example, CHO cells; or in a viral system, such as, for example, bacculovirus.

In a second embodiment, SOD is of natural origin. Advantageously, the composition comprises a natural extract including SOD, preferably the natural extract is a vegetal extract. In an embodiment, the vegetal extract comprising SOD is not from a genetically modified organism (GMO) or genetically engineered organism (GEO). According to the invention, said vegetal extract is selected from the group comprising Red Seaweed, Green Seaweed, Watermelon (*Citrullus vulgaris* Schrad) cotyledons, Oats, Peas, Com and Melon. In a preferred embodiment, said vegetal extract is from melon, preferably from *Cucumis melo* LC. (Cucurbitaceae).

In a first embodiment of the invention, the amount of vegetal extract including SOD in the composition ranges from 10 to 500 mg, preferably from 50 to 200 mg, more preferably is about 100 mg. In this embodiment, preferably the percentage of vegetal extract including SOD ranges from 50-95% in weight to the weight of all active agents present in the composition.

In a second embodiment of the invention, the composition is such that a daily amount of vegetal extract including SOD ranging from 10 to 500 mg, preferably from 50 to 200 mg, more preferably of about 100 mg is provided.

In a third embodiment of the invention, the amount of vegetal extract including SOD in the composition ranges from 50 to 2000 mg, preferably from 100 to 1000 mg, more preferably of about 250 mg. In this embodiment, preferably the percentage of vegetal extract including SOD ranges from 65-95% in weight to the weight of all active agents present in the composition. Preferably, the percentage of vegetal extract including SOD ranges from 30-50% in weight to the total weight of the composition.

In a fourth embodiment of the invention, the composition is such that a daily amount of vegetal extract including SOD ranging from 100 to 5000 mg, preferably from 200 to 2000 mg, more preferably from 250 to 1000 mg is provided.

In a preferred embodiment, the enzymatic activity of SOD is of at least 0.5 UI/mg, preferably of at least 1 UI/mg, more preferably of at least 1.1 UI/mg.

Superoxide dismutase is a metalloprotein, which exists in several forms, dependant on the metal present in the molecule (Zn, Cu, Fe, Mn or Ni). The two principal forms of SOD are Cu/Zn SOD and Mn SOD. The first one, Cu/Zn SOD, comprises two Cu²⁺ atoms and two Zn²⁺ atoms per molecule of enzyme, whereas the second one, Mn SOD, comprises Mn²⁺ atoms. Cu/Zn SOD shows the highest efficiency to counteract oxidation.

In an embodiment of the invention, SOD is Cu/Zn SOD and/or Mn SOD. In a preferred embodiment, SOD is Cu/Zn SOD.

SOD is a protein, and is thus degraded after oral ingestion, in the stomach and during digestion. In order to protect SOD against this degradation, SOD may be protected by micro-encapsulation. Injection or ingestion of micro-encapsulated elements shall lead to the sustained release of SOD at the desired site, preventing any prior degradation of said element by the digestive apparel. Micro-encapsulation may be based on polymerization or reticulation.

According to the invention, SOD is micro-encapsulated.

In a first embodiment, SOD is micro-encapsulated in a biopolymer, preferably made of prolamin. The presence of prolamin may retain the active ingredient and delay its release in the small intestine. Advantageously, prolamin is extracted from a vegetal selected from the group comprising, but not limited to, rye (secalin), wheat (gliadin), oats (avenin), barley (hordein), barley herb, rice, birdseed and corn. In a preferred embodiment, said prolamin is gliadin, preferably extracted from wheat, more preferably wheat is selected from the group comprising *Triticum vulgare, Triticum durum, Triticum herbe, Triticum spelta* and *Triticum polonicum,* even more preferably wheat is *Triticum vulgare.* According to an embodiment, gliadin from *Triticum vulgare* is used in its native form, i.e. non denaturized. Wheat gliadin is commercially available. In an embodiment, wheat gliadin is Gliamine® from HITEX (Vannes, France). In a preferred embodiment, SOD micro-encapsulated by gliadin is GliSODin®, provided by Isocell (Paris, France). GliSODin® is a mix of Extramel® (from Bionov company) with gliadin and maltodextrine. Extramel® is an extract from *Cucumis melo* LC., that comprises 3 to 7-fold more SOD than other melon species. In an embodiment, prolamin may be provided in the form of a prolamin based peptide fragment, preferably a gliadin based peptide fragment. A gliadin based peptide fragment is a fragment of gliadin or a derivative, analog, salt or metabolite thereof. More preferably, prolamin based peptide fragment is a non-immunogenic analog of gliadin. One prolamin based peptide fragment is chosen from the group consisting of fully hydrolyzed, substantially hydrolyzed or slightly hydrolyzed prolamin based peptide fragments.

In a second embodiment, SOD is micro-encapsulated with a biopolymer made of poly(cyclohexane-1,4-diyl acetone dimethyl ketal) (PCADK).

According to an embodiment, the micro-encapsulated SOD is further coated with a polymer, which facilitates its digestive absorption and/or enhances its protection from degradation. In an embodiment, said polymer is selected from the group comprising povidone, chitosan, hyaluronic acid, polysome, phytoliposome, dextran, titanium dioxide, polysaccharide, lipids, phosphatidylcholine. In a preferred embodiment, said polymer is chitosan and/or hyaluronic acid.

In an embodiment, said particles of the micro-encapsulated SOD have a size ranging from 200 nM to 1000 µM.

In an embodiment of the invention, the at least one zeaxanthin isomer is selected from the group consisting of (3R, 3'R) zeaxanthin, (3R, 3'S) zeaxanthin (mesozeaxanthine) and (3S, 3'S) zeaxanthine isomer; preferably, the zeaxanthin isomer is zeaxanthin and/or mesozeaxanthin. Mesozeaxanthin is a stereo-isomer of zeaxanthin, synthesized in the eye from lutein. It might be more efficient than lutein for essential activities in the center of the macula.

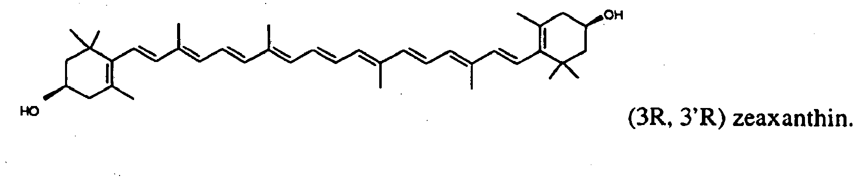

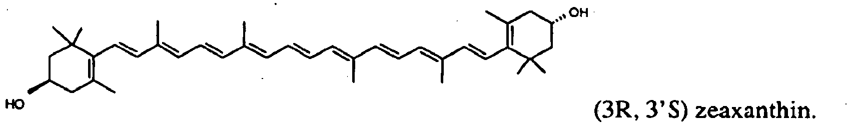

In a preferred embodiment, said composition comprises lutein and at least one zeaxanthin isomer, present in a lutein:zeaxanthin isomer ratio of about 5:1. Preferably, the lutein and at least one zeaxanthin isomer are extracted from *Tagetes erecta,* and are free from pesticides, dioxin and genetically modified organism (GMO) or genetically engineered organism (GEO). More preferably, the lutein and at least one zeaxanthin isomer are in a crystalline form, such as, for example, lutein/zeaxanthin FloraGlo®, commercially available from LCF.

In an embodiment, lutein is provided as lutein free 20%. In still another embodiment, zeaxanthin isomer is provided as zeaxanthin 20%. Preferably, lutein and zeaxanthin isomer are provided as concentrated oleoresin from *Tagetes erecta* flowers, preferentially titrated respectively at 200 mg of lutein/g and 200 mg of zeaxanthin/g, respectively.

In a first embodiment of the invention, the amount of lutein in the composition ranges from 1 to 10 mg, preferably from 2.5 to 7.5 mg, more preferably is about 5 mg. Preferably, lutein ranges from 10-30% in weight to the weight of active agents in the composition.

In a second embodiment of the invention, the composition is such that a daily amount of lutein ranging from 1 to 10 mg, preferably from 2.5 to 7.5 mg, more preferably of about 5 mg is provided.

In a third embodiment of the invention, the amount of lutein in the composition ranges from 1 to 25 mg, preferably from 3 to 15 mg, more preferably is about 5 mg. Preferably, lutein ranges from 3-5% in weight to the weight the composition.

In a fourth embodiment of the invention, the composition is such that a daily amount of lutein ranging from 2 to 100 mg, preferably from 5 to 50 mg, more preferably from 10 to 20 mg is provided.

In a first embodiment of the invention, the amount of at least one zeaxanthin isomer in the composition ranges from 0.1 to 2 mg, preferably from 0.5 to 1.5 mg, more preferably is about 1 mg. Preferably, zeaxanthin ranges from 1.5-8% in weight to the weight of active agents in the composition.

In a second embodiment of the invention, the composition is such that a daily amount of at least one zeaxanthin isomer ranging from 0.1 to 2 mg, preferably from 0.5 to 1.5 mg, more preferably of about 1 mg is provided.

In a third embodiment of the invention, the amount of at least one zeaxanthin isomer in the composition ranges from 0.25 to 5 mg, preferably from 1 to 3 mg, more preferably is about 1 mg. Preferably, zeaxanthin ranges from 0.6-1% in weight to the weight the composition.

In a fourth embodiment of the invention, the composition is such that a daily amount of at least one zeaxanthin isomer ranging from 1 to 3, preferably from 1.5 to 2.5, more preferably of about 2 mg, is provided.

In an embodiment of the invention, lutein and zeaxanthin isomer are micro-encapsulated with a biopolymer, preferably made of prolamin. In another embodiment, lutein and zeaxanthin isomer is micro-encapsulated with a biopolymer made of PCADK.

In an embodiment of the invention, lutein, zeaxanthin isomer and SOD are micro-encapsulated in the same particles. According to another embodiment, lutein and zeaxanthin isomer are further coated with a polymer, to facilitate their digestive absorption. In an embodiment, said polymer is chitosan and/or hyaluronic acid. According to an embodiment, the lutein, at least one zeaxanthin isomer and SOD are present in the same chitosan and/or hyaluronic acid coated particles.

According to an embodiment, the composition of the invention further comprises one or more other non-enzymatic antioxidant agent, selected from the group comprising vitamins, such as, for example, Vitamin A, Vitamin C, Vitamin E or Vitamin B19; minerals such as, for example, copper, manganese, zinc or selenium; vegetal antioxidants such as, for example, anthocyanosids, glucosinolates, lycopene or polyphenols such as, for examples, flavonoids, catechins, stilbenoids, isoflavones; Coenzyme Q10 and poly-unsaturated lipid, such as, for example, omega3.

According to an embodiment the composition further comprises excipients, such as for example coloring agents, flavoring agents, thickeners, such as, for example, glycerol monostearate, sweeteners, coating agents, such as, for example, refined colza oil, soya oil, peanut oil, soya lecithin or fish gelatin, diluting agents, such as, for example, lactose, monohydrated lactose or starch, binding agents, such as, for example, povidone, pregelatinized starch, gums, saccharose, polyethylene glycol (PEG) 4000 or PEG 6000, disintegrating agents, such as, for example, microcrystalline cellulose or sodium carboxymethyl starch, such as, for example, sodium carboxymethyl starch type A, lubricant agents, such as, for example, magnesium stearate, flow agent, such as, for example, colloidal anhydrous silica, etc.... Advantageously, the above-mentioned excipients are present in an amount ranging from 50 to 80% in weight to the total weight of the composition.

According to an embodiment, the composition of the invention is an intimate mixture of SOD with lutein and at least one zeaxanthin isomer. In a further embodiment, the composition consists of SOD with lutein and at least one zeaxanthin isomer and suitable excipients.

According to an embodiment, the composition of the invention is to be orally administered.

According to the invention, the composition may be in a solid form (pill, tablet, capsule, soft gelatin capsule, sugar-coated pill, orodispersing/orodisintegrating tablet, powder, effervescent tablet, etc...), in a liquid form (injectable solution, drinkable solution, buccal spray, eye drops, ointment...), or in the form of a gel.

The invention also relates to a kit of parts comprising the composition of the invention, and preferably comprising two parts. According to an embodiment, a first part of the kit comprises SOD, optionally protected by a polymer such as, for example prolamin, and a second part comprises lutein and at least one isomer of zeaxanthin. According to an embodiment, both parts of the kit are orally administered. In an embodiment, each part of the kit is independently in a solid form (pill, tablet, capsule, soft gelatin capsule, sugar-coated pill, orodispersing/orodisintegrating tablet, powder, effervescent tablet, etc...), in a liquid form (injectable solution, drinkable solution, buccal spray, eye drops, ointment ...), or in the form of a gel.

According to an embodiment, one of the first and second parts of the kit is formulated for sustained release thereof. In an embodiment, when formulated for sustained release thereof, the part of the kit is in a solid form, preferably is a coated pill, a coated tablet, a soft gelatin tablet or a pill with modified release kinetic. When the part of the kit is not formulated for sustained release thereof and is in a solid form, it is a non-coated pill, a non-coated tablet, a soluble pill, a dispersible pill or an orodispersible pill.

According to a first embodiment of the invention, the first and second parts of the kit of parts are administered simultaneously to a subject. According to a preferred embodiment, both parts of the kit are administered once a day, preferably in the morning.

According to a second embodiment of the invention, the first and second parts of the kit are administered sequentially to a subject. According to this embodiment, the first part comprising SOD is administered 12 hours before or after the second part comprising lutein and at least one zeaxanthin isomer. According to a preferred embodiment, the first and second parts of the kit are administered once a day, preferably the first one in the morning and the second one in the evening.

The invention also relates to a functional food, a nutraceutical composition or a food or dietary supplement, comprising the composition of the invention, or the kit of parts of the invention, as previously described. According to the invention, the functional food, the nutraceutical composition or the food or dietary supplement of the invention is for maintaining visual health and improving visual comfort.

According to an embodiment, the functional food, nutraceutical composition or food or dietary supplement is suitable for oral administration, and is preferably in a solid form, in a liquid form or in the form of a gel.

According to an embodiment, the functional food, nutraceutical composition or food or dietary supplement is to be ingested once a day, during a period of time ranging from 1 to 120 days, preferably from 10 to 50 days, more preferably during about 30 days.

According to a first embodiment, the functional food, nutraceutical composition or food or dietary supplement is recommended to a subject with a genetic predisposition to or a risk of developing an ocular disease, disorder or condition related to oxidative stress, such as, for example, Age-related Macular Degeneration (AMD), diabetic retinopathies, cataract and retinitis pigmentosa.

According to a second embodiment, the functional food, nutraceutical composition or food or dietary supplement is recommended to a subject exposed to environmental stress, such as, for example, tobacco, air pollution, UV light and the like causing oxidative stress to the eye. The composition of the invention may be a visual care product, which means that it maintains visual health.

According to a third embodiment, the functional food, nutraceutical composition or food or dietary supplement is for use in preventing ocular aging.

The invention also relates to a pharmaceutical composition comprising the composition of the invention, or the kit of parts of the invention, as previously described, in association with at least one pharmaceutically acceptable excipient. The invention also relates to a medicament comprising the composition of the invention, or the kit of parts of the invention, as previously described.

According to a first embodiment, the pharmaceutical composition or the medicament of the invention is orally administered. Advantageously, the medicament or the pharmaceutical composition is in a solid form, in a liquid form or in the form of a gel.

According to a second embodiment, the medicament or the pharmaceutical composition of the invention is topically administered, preferably is applied topically on the eye surface. Advantageously, the medicament or the pharmaceutical composition is an eye drop, an ointment and the like.

According to a third embodiment, the medicament or the pharmaceutical composition of the invention is intraocularly administered. By intraocularly is meant an injection directly in the eye ball, preferably inside the vitreous chamber.

According to a fourth embodiment, the medicament or the pharmaceutical composition of the invention is injected, preferably systemically injected.

Advantageously, when injected, the composition comprises recombinant SOD.

According to the invention, the pharmaceutical composition or the medicament of the invention is useful for preventing, stabilizing or treating a retinal pathology. In an embodiment, said retinal pathology is associated with oxidative stress, such as, for example. Age-related Macular Degeneration (AMD), diabetic retinopathies, cataract and retinitis pigmentosa. According to an embodiment, the composition of the invention is for preventing and/or treating ocular ageing.

According to an embodiment, the composition is for use before or after surgery of cataract.

According to another embodiment, the pharmaceutical composition of the invention or the medicament of the invention is administered 1 to 5 times, preferably from 2 to 4 times a day, during a recommended period of treatment of at least 1 to 3 weeks. Such treatment may be repeated one to three times. In severe pathologies, the recommended period of treatment may be indefinite, which means that the treatment may be a chronic treatment.

The present invention also relates to a veterinarian product, comprising the functional food, nutraceutical composition or food or dietary supplement, or the pharmaceutical composition of the invention.

In a preferred embodiment, the composition of the invention is administered to a subject in need thereof, through an oral way. According to an embodiment, the subject is an animal, preferably a mammal (cat, dog, hamster, rabbit, mouse, gerbil, rat, Guinea pig, human...), more preferably a human.

The invention also relates to preventing, stabilizing or treating a retinal pathology, preferably a retinal pathology associated with oxidative stress, comprising administering to a subject in need thereof a composition or a kit of parts comprising SOD, preferably SOD micro-encapsulated with gliadin, lutein and at least one zeaxanthin isomer.

### BRIEF DESCRIPTION OF THE DRAWINGS

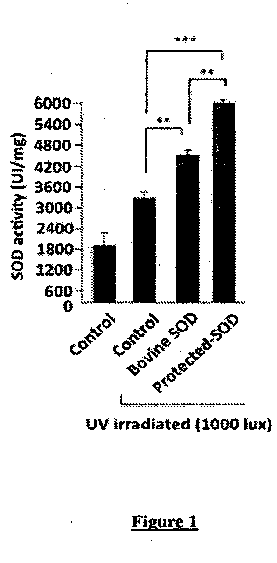

**Figure 1** is a histogram showing a comparison of Bovine and vegetal protected SOD on UV-irradiated RPE. SOD activity was measured in RPE stimulated by UV (1000 lux) treated with excipient (control), Bovine SOD origin (SIGMA®) or protected-vegetal SOD origin compared to non-irradiated RPE. Values are means±SD. **p < 0.01, ***p < 0.001.

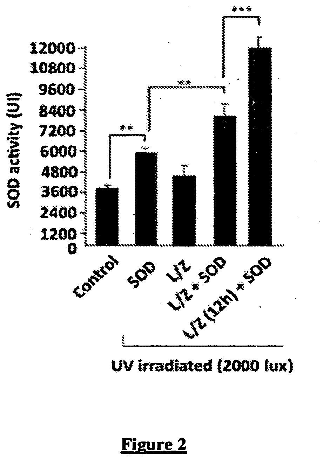

**Figure 2** is a histogram showing the plasmatic c-SOD (cellular-SOD) activity on UV-irradiated mice. Non-UV irradiated mice, n=10; UV-irradiated mice, n=12; UV-irradiated mice fed lutein/zeaxanthine (L/Z) diet, n=5; UV-irradiated mice fed SOD diet, n=10; UV-irradiated mice fed lutein/zeaxanthine + SOD diet, n=15; UV-irradiated mice fed lutein/zeaxanthine 12 hours before SOD diet, n=15. Values are means±SD. **p < 0.01, ***p < 0.001.

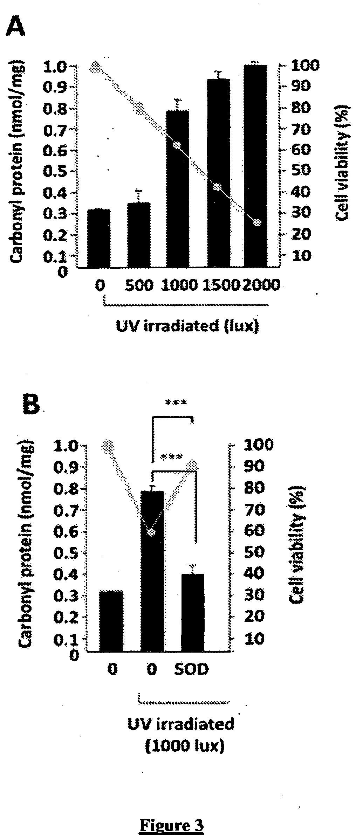

**Figure 3** is a combination of two histograms, showing the effect SOD on RPE cells irradiated with UV. A- UV-irradiation increased carbonyl protein quantity on RPE cells in a dose dependent of lux intensity (black histograms). In correlation with oxydative stress and carbonyl protein concentration, UV induced cell death (grey curve). **B-** SOD exposure reversed apoptosis induced by UV in RPE. Values are means±SD. **p < 0.01, ***p < 0.001.

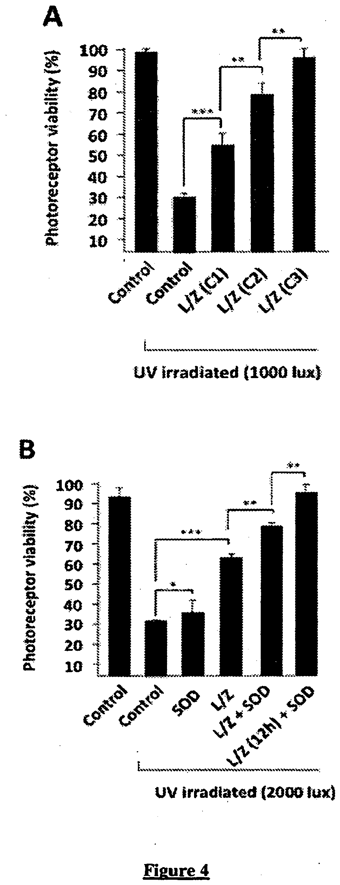

**Figure 4** is a combination of two histograms, showing the effect of lutein and zeaxanthin on the viability of UV-irradiated photoreceptors. UV-irradiation increased photoreceptor apoptosis in cell culture (**A**) and *in vivo* (**B**) or in retina culture explants (i.e. two *in vivo* results, data not show). Lutein/zeaxanthin decreased photoreceptor apoptosis in a dose dependent manner (concentration; C1 to C3). Cell viability was assessed using DHE and AnnexinV/PI stainings. Values are means±SD. *p < 0.5. **p < 0.01, ***p < 0.001.

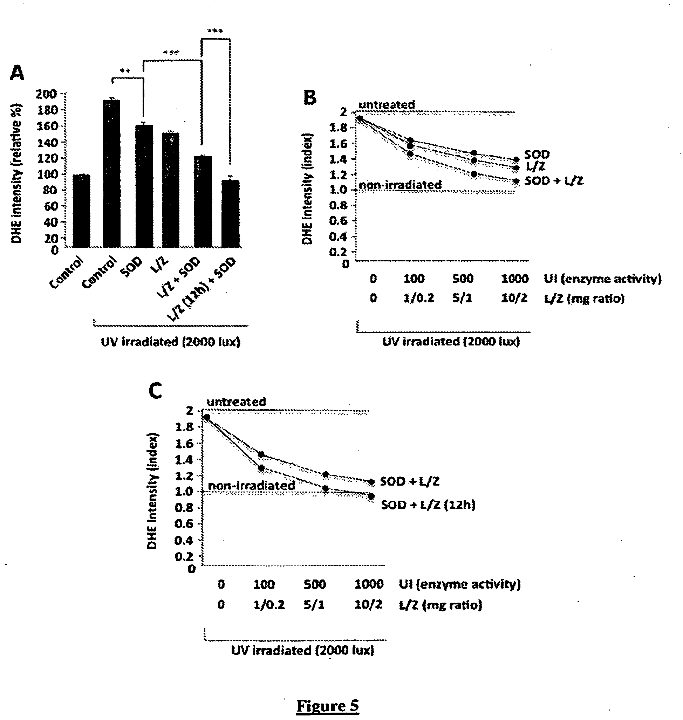

**Figure 5** is a combination of graphs, showing that UV-induced oxidative stress in the retina is reduced by the addition of both lutein/zeaxanthine and SOD. The fluorescence intensity in the inner nuclear layer relative to that of un-irradiated mice was measured by the Image J program. A- Non-UV irradiated mice, n=10; UV-irradiated mice, n=12; UV-irradiated mice fed lutein/zeaxanthine (L/Z) diet, n=5; UV-irradiated mice fed SOD diet, n=10; UV-irradiated mice fed lutein/zeaxanthine + SOD diet, n=15; UV-irradiated mice fed lutein/zeaxanthine 12 hours before SOD diet, n=15; in a lutein/zeaxanthin and SOD dose dependence (**B**) and efficiency increased with 12 hours pre-treatment with lutein/zeaxanthin (C). Values are means±SD. **p < 0.01, ***p < 0.001.

### EXAMPLES

The present invention is further illustrated by the following examples.

### Example 1:

### Nutraceutical product (per unitary dosage)

| **Active agents** | **Weight (mg)** |
|---|---|
| SOD | 50-200, preferably 100 |
| Lutein | 2.5-7.5, preferably 5 |
| Zeaxanthin | 0.5-1.5, preferably 1 |
| Excipients | 53-350, preferably 150 |

### Example 2:

### Nutraceutical product (per unitary dosage - preferred unitary dosage form is a capsule)

| **Ingredients** | | **Quantity (mg)** | **Dosage per capsule (mg)** |
|---|---|---|---|
| Active agents | SOD (GliSODin® from Isocell) | 50-200 | 100 |
| | Lutein free 20 % | 12.5-37.5 *Equivalent 2.5-7.5 lutein* | 26.3 *Equivalent 5.26 lutein* |
| | zeaxanthin free 20 % | 2.5-7.5 *Equivalent 0.5-1.5 zeaxanthin* | 5.0 *Equivalent 1 zeaxanthin* |
| | *Total of active agents* | *65-245* | *131.3* |
| Excipients | Refined colza oil | 200-250 | 230 |
| | Fish gelatin | 50-75 | 63.9 |
| | Glycerol monostearate (E471 - thickener) | 10-30 | 19.0 |
| | Water | 5-15 | 10.8 |
| | Colouring agents | 0.1-10 | 1.3 |
| | *Total of excipients* | *265.1-380* | *325* |

### Nutraceutical product (in percentage* per unitary dosage - preferred unitary dosage form is a capsule)

| **Ingredients** | | **Range of Percentage** | **Preferred percentage** |
|---|---|---|---|
| Active agents | SOD (GliSODin® from Isocell) | 50-95 | 76.2 |
| | Lutein free 20 % | 10-30 | 20.0 |
| | zeaxanthin free 20 % | 1.5-8 | 3.8 |
| | *Total of active agents* | *100* | *100* |
| Excipients | Refined colza oil | 50-90 | 70.8 |
| | Fish gelatin | 10-30 | 19.7 |
| | Glycerol monostearate (E471 - thickener) | 0-10 | 5.8 |
| | Water | 0-10 | 3.3 |
| | Colouring agents | 0-5 | 0.4 |
| | *Total of excipients* | *100* | *100* |

| | | | |
|---|---|---|---|
| *: percentages are in weight to the total weight of active ingredients or excipients respectively. | | | |

### Example 3:

### Pharmaceutical composition (tablet)

| **Ingredients** | | **mg** | **Percentage*** |
|---|---|---|---|
| Active agents | SOD (GliSODin® from Isocell) | 250.00 mg | 35.6 % |
| | Lutein free 20 % | 25.00 mg | 3.6 % |
| | Zeaxanthin 20 % | 5.00 mg | 0.7 % |
| Excipients | Monohydrated Lactose | 299.00 mg | 42.7 % |
| | Povidone | 14.00 mg | 2.0 % |
| | Microcrystalline Cellulose | 74.00 mg | 10.6 % |
| | Sodium carboxymethyl starch (type A) | 28.00 mg | 4.0 % |
| | Magnesium stearate | 5.50 mg | 0.8 % |
| | **TOTAL** | **700.5 mg** | **100.0 %** |

| | | | |
|---|---|---|---|
| *: percentages are in weight to the total weight of the tablet. | | | |

### Example 4:

### Pharmaceutical composition (capsule, size 0)

| **Ingredients** | | **mg** | **Percentage*** |
|---|---|---|---|
| Active agents | SOD (GliSODin® from Isocell) | 250.00 mg | 33.5 % |
| | Lutein free 20 % | 25.00 mg | 3.3 % |
| | Zeaxanthin 20 % | 5.00 mg | 0.7 % |
| Excipients | Monohydrated lactose | 294.00 mg | 39.4 % |
| | Pregelatinized starch | 158.5 mg | 21.3 % |
| | Colloidal anhydrous silica | 6.6 mg | 0.9 % |
| | Magnesium stearate | 6.6 mg | 0.9 % |
| | **TOTAL** | **745.7 mg** | **100.00 %** |

| | | | |
|---|---|---|---|
| *: percentages are in weight to the total weight of the capsule. | | | |

### Example 5:

### Pharmaceutical composition (soft gelatin capsule)

| **Ingredients** | | **mg** | **Percentage*** |
|---|---|---|---|
| Active agents | SOD (GliSODin® from Isocell) | 250.00 mg | 43.1 % |
| | Lutein free 20 % | 25.00 mg | 4.3 % |
| | Zeaxanthin 20 % | 5.00 mg | 0.9 % |
| Excipients | Peanut oil | 298.00 mg | 51.4 % |
| | Soya lecithin | 2.00 mg | 0.3 % |
| | **TOTAL** | **580.00 mg** | **100.00 %** |

| | | | |
|---|---|---|---|
| *: percentages are in weight to the total weight of the soft gelatin capsule. | | | |

### Example 6:

### Materials and Methods

### Material

When not specified, SOD is a vegetal extract containing SOD associated to gliadin to protect it and contribute to its intestinal absorption. 1 mg of SOD/gliadin mix has at least a SOD activity of 1.1 UI/mg.

### Methods

- Animals: C57BL/6 mice were UV exposed (2000 lux during 5 hours). Mice fed a lutein/zeaxanthin (1 to 10 mg range), SOD (100 to 1000 unit range) or a mix of both-supplemented.
- Retina explants: The eyes were enucleated and placed in a dish containing Nutrient medium R16 with the addition of 10% fetal calf serum (Gibco), 2.56 mg/L cytidine 5-diphosphocholine, 1.28 mg/L cytidine diphosphoethanolamine, used as culture medium. An eyeball incision was made behind the limbus and the cornea-iris complex, lens and vitreous were removed. The explants were placed lying flat on a 10 mm nitrocellulose membrane (Millipore, pore size 0.45 pm) with the vitreal side facing upwards. The culture dishes were placed in an incubator with 5% C02 in air, 100% humidity at 37° Celsius.
- Cell cultures: Briefly, human cells from retina (Retinal pigment epithelial - RPE cells) ARPE-19 type (ATTC Ref. CRL-2302) were used for culture experiments. RPE cells were seeded at 10⁶ cells/mL and plated at 37°C, 5% CO2, using Dulbecco's medium (Invitrogen®) supplemented with 10% SVF, 1.2 g/L sodium bicarbonate, 2.5 mM L-glutamine, 15 mM HEPES and 0.5 mM sodium pyruvate. Mice retinas were dissected and dissociated by trypsin digestion followed by mechanical dissociation. After dissociation, the cells were cultured in photoreceptor-defined medium. Approximately 10⁶ cells were seeded on 35-mm diameter dishes. Cultures were incubated at 36°C in a humidified atmosphere of 5% CO2.
- SOD activity measurement *in vitro:* SOD activity is measured using RANSOD KIT (RANDOX®) in ARPE-19 (Retinal pigment epithelial - RPE cells) (ATTC Ref. CRL-2302) under treatment conditions. Cells are cultured at 10⁶ cells/ml in 6 well plates (NUNC®) at 37°C, 5% CO₂, using Dulbecco's medium (INVITROGEN®) supplemented with 10% SVF, 1.2 g/L sodium bicarbonate, 2.5 mM L-glutamine, 15 mM HEPES, 0.5 mM sodium pyruvate. After treatment, cells were harvested using trypsine and lysed by sonication. Protein extracts are quantified, and SOD activity is measured following RANDOD KIT instruction. Results are expressed in SOD activity (UI)/mg of protein ratio.
- SOD activity measurement *in vivo:* Briefly, blood samples (0.5 ml) are collected from mice exposed to UV irradiation treated or not with SOD, lutein/zeaxanthin (L/Z), SOD + L/Z or with time delay (12 hours) of L/Z and SOD administration. Blood samples are centrifuged at 1000g, 30 minutes at 4°C, erythrocyte pellets are reconstituted and washed using 0.5 ml of 0,172 mol/L (pH=7.6) Tris HCl buffer. Erythrocytes are lysed using 3 ml of 0.011 ml/L (pH=7.6) Tris HCl buffer, and membranes are separated using 2000 g, 40 minutes centrifugation procedures. Membrane pellets are washed using 0.011 ml/L (pH=7.6) Tris HCl buffer (3 times) and SOD activity is measured using RANSOD KIT (RANDOX®) as previously described.
- Biotin-dUTP Nick-end Labeling (TUNEL): Apoptosis was examined by TUNEL assay (DeadEnd Fluorometric TUNEL system, Promega, Madison, WI). Sections were counterstained with 4,6-diamidino-2-phenylindole (DAPI) after the reaction. Only sections with optic nerve stumps were used and images were captured from the central, the middle, and from the peripheral retina. The number of TUNEL-positive nuclei in both ganglion cell layer and inner nuclear layer were counted.
- ROS measurement in retina: Eyes were enucleated and immediately frozen in OCT compound (Sakura Finetek, Torrance, CA, USA). Retina explants were also fixed in OCT. Unfixed cryosections (10 µm) using a microtome (Microm HM 315R, Heidelberg, Germany), were incubated with dihydroethidium (DHE; Invitrogen-Molecular Probes, Eugene, OR, USA) (5 µmol/L) for 20 min at 37°C. DHE specifically reacts with intracellular superoxide anion and is converted to the red fluorescent compound ethidium in nuclei. The sections were examined using a microscope possessing a digital camera (Carl Zeiss, Jena, Germany), and the intensity of the staining was measured in the posterior retina, using the Image J software (National Institutes of Health, Bethesda, MD, USA).
- Apoptosis assay: For TUNEL staining, the cells were fixed with 2% PFA for 15 minutes and stored in 70% ethanol for 48 hours at -20°C. Before labeling, cells were washed twice with PBS for 5 minutes at room temperature. Samples were incubated with TdT reaction mixture (0.05 mM BrdUTP, 0.3 U/µL TdT) at 37°C for 1 hour. The reaction was stopped using buffer (300 mM NaCl, 30 mM sodium citrate; pH=7.4) at room temperature. Negative controls were prepared by omitting TdT. BrdU was detected using an anti-BrdU monoclonal antibody and Annexin V staining was performed. The cells were washed twice in cold PBS, labeled with Annexin V/PI buffer and were then analyzed by fluorescence.

### Results

*Vegetal protected SOD is more efficient than Bovine SOD to stimulate SOD activity in UV-irradiated RPE cells.* The treatment of UV-irradiated RPE cells by SOD enhances measured SOD activity (in UI/mg). Moreover, vegetal SOD protected with gliadin is more efficient than naked bovine SOD (Figure 1).

*Effects of SOD and*/*or lutein*/*zeaxanthin on c-SOD activity:* As previously shown *in vitro* (see above and figure 1), SOD also enhances measured c-SOD activity *in vivo* in irradiated mice. In parallel, the treatment of UV-irradiated mice by lutein/zeaxanthin slightly stimulates c-SOD activity. SOD and lutein/zeaxanthin exhibit a synergic effect on the stimulation of c-SOD activity *in vivo.* Surprisingly, the synergistic effect is enhanced when both products are administered with a 12 hours delay (figure 2).

### Protection of cells from oxidative damages by SOD and/or lutein/zeaxanthin:

UV-irradiation of RPE cells leads to oxidative damages, such as protein carbonylation and to a decrease in cell viability. The treatment of cultured RPE cells with SOD preserves those cells from such damages (figure 3).
SOD and lutein/zeaxanthin (dose dependent effect) preserve photoreceptor viability after UV-irradiation, both *in vivo* and *ex vivo.* Both products show a synergic effect, which is enhanced when they are administered with a 12 hours delay (figure 4).

SOD and lutein/zeaxanthin preserve *in vivo* the retina from oxidative stress (measured by DHE intensity). Both products still show a synergic effect, which is enhanced when they are administered with a 12 hours delay (figure 5).

## Claims

1. A composition comprising superoxide dismutase (SOD) in association with lutein and at least one zeaxanthin isomer.

2. The composition according to of claim 1, wherein the SOD is of natural origin, and the composition comprises a vegetal extract including SOD, preferably an extract of *Cucumis melo* LC, preferably the amount of vegetal extract including superoxide dismutase ranges from 10 to 2000 mg, preferably from 50 to 1000 mg, more preferably 100-500 mg.

3. The composition according to claim 1 or claim 2, wherein enzymatic activity of said SOD is of at least 1.1 UI/mg.

4. The composition according to anyone of claims 1 to 3, wherein SOD is micro-encapsulated with a biopolymer, preferably a prolamine or PCADK.

5. The composition according to claim 4, wherein the micro-encapsulated SOD is further coated with a polymer, preferably chitosan or hyaluronic acid.

6. The composition according to anyone of claims 1 to 5, wherein the at least one zeaxanthin isomer is selected from the group consisting of zeaxanthin and mesozeaxanthine.

7. The composition according to anyone of claims 1 to 6, wherein the amount of lutein ranges from 0.5 to 25 mg, and the amount of at least one zeaxanthin isomer ranges from 0.1-5 mg.

8. The composition according to anyone of claims 1 to 7, wherein the lutein and at least one zeaxanthin isomer are present in the composition in a ratio lutein:zeaxanthin isomer of about 5:1.

9. The composition according to anyone of claims 1 to 8, wherein the association is an intimate mixture of superoxide dismutase, lutein and at least one zeaxanthin isomer.

10. A kit of parts comprising a composition according to anyone of claims 1 to 9, wherein the kit comprises a first part comprising superoxide dismutase, and a second part comprising lutein and at least one zeaxanthin isomer.

11. The kit according to claim 10, wherein one of the first and second part is formulated for sustained release thereof.

12. The kit according to claim 10 or claim 11, wherein the first and second parts are to be administered to an individual simultaneously or sequentially, and when administered sequentially, the first part comprising SOD is to be administered 12 hours before or after the second part comprising lutein and at least one zeaxanthin isomer.

13. A functional food, a nutraceutical composition or a food or dietary supplement comprising a composition according to anyone of claims 1 to 9 or a kit of parts according to anyone of claims 10 to 12.

14. The functional food, the nutraceutical composition or the food or dietary supplement according to claim 13, comprising 50-200 mg, preferably about 100 mg SOD, 2.5-7.5 mg, preferably about 5 mg lutein and 0.5-1.5 mg, preferably about 1 mg zeaxanthin.

15. The functional food, the nutraceutical composition or the food or dietary supplement according to claim 13 or claim 14, in a form suitable for being orally administered.

16. A pharmaceutical composition comprising a composition according to anyone of claims 1 to 9 or a kit of parts according to anyone of claims 10 to 12 in association with at least one pharmaceutically acceptable excipient.

17. A medicament comprising a composition according to anyone of claims 1 to 9 or a kit of parts according to anyone of claims 10 to 12.

18. The pharmaceutical composition according to claim 16 or the medicament of claim 17, comprising 100-1000 mg superoxide dismutase, 1-25 mg lutein and 0.5-5 mg zeaxanthin.

19. The pharmaceutical composition or the medicament according to anyone of claims 16 to 18, in a form suitable for being administered orally, topically, intraocularly or systemically.

20. A veterinarian product, comprising a composition according to anyone of claims 1 to 9 or a kit of parts according to anyone of claims 10 to 12.

## Patentansprüche

1. Zusammensetzung, umfassend Superoxiddismutase (SOD) in Verbindung mit Lutein und mindestens einem Zeaxanthinisomer.

2. Zusammensetzung nach Anspruch **1,** wobei SOD natürlichen Ursprungs ist und die Zusammensetzung einen pflanzlichen Extrakt umfasst, darin eingeschlossen SOD, vorzugsweise einen Extrakt von *Cucumis melo* LC, wobei vorzugsweise die Menge des pflanzlichen Extrakts, darin eingeschlossen Superoxiddismutase, zwischen 10 und 2000 mg liegt, vorzugsweise zwischen 50 und 1000 mg, insbesondere zwischen 100 und 500 mg.

3. Zusammensetzung nach Anspruch **1** oder Anspruch **2,** wobei die enzymatische Aktivität von SOD mindestens 1,1 Ul/mg beträgt.

4. Zusammensetzung nach einem der Ansprüche **1** bis **3,** wobei SOD mit einem Biopolymer mikroverkapselt ist, vorzugsweise mit einem Prolamin oder PCADK.

5. Zusammensetzung nach Anspruch **4,** wobei die mikroverkapselte SOD weiter mit einem Polymer beschichtet ist, vorzugsweise Chitosan oder Hyaluronsäure.

6. Zusammensetzung nach einem der Ansprüche **1** bis **5,** wobei das mindestens eine Zeaxanthinisomer ausgewählt ist aus der Gruppe bestehend aus Zeaxanthin und Mesozeaxanthin.

7. Zusammensetzung nach einem der Ansprüche **1** bis **6,** wobei die Menge von Lutein zwischen 0,5 und 25 mg liegt, und die Menge mindestens eines Zeaxantinisomers zwischen 0,1 und 5 mg liegt.

8. Zusammensetzung nach einem der Ansprüche **1** bis **7,** wobei das Lutein und mindestens ein Zeaxanthinisomer in der Zusammensetzung in einem Verhältnis Lutein:Zeaxanthinisomer von ungefähr 5:1 vorhanden sind.

9. Zusammensetzung nach einem der Ansprüche **1** bis **8,** wobei die Verbindung eine innige Mischung aus Superoxiddismutase, Lutein und mindestens einem Zeaxantinisomer ist.

10. Satz von Teilen, umfassend eine Zusammensetzung nach einem der Ansprüche **1** bis **9,** wobei der Satz einen ersten Teil umfasst, umfassend Superoxiddismutase, und einen zweiten Teil, umfassend Lutein und mindestens ein Zeaxanthinisomer.

11. Satz nach Anspruch **10,** wobei einer des ersten Teils und des zweiten Teils für eine verzögerte Freisetzung davon formuliert ist.

12. Satz nach Anspruch **10** oder Anspruch **11,** wobei der erste und der zweite Teil einem Individuum gleichzeitig oder sequentiell verabreicht werden müssen, und wenn sie sequentiell verabreicht werden, der erste Teil, umfassend SOD, 12 Stunden vor oder nach dem zweiten Teil, umfassend Lutein und mindestens ein Zeaxanthinisomer, verabreicht werden muss.

13. Funktionelles Nahrungsmittel, nutrazeutische Zusammensetzung oder Nahrungsmittel- oder Diätzusatz, umfassend eine Zusammensetzung nach einem der Ansprüche 1 bis 9 oder Satz von Teilen nach einem der Ansprüche **10** bis **12**.

14. Funktionelles Nahrungsmittel, nutrazeutische Zusammensetzung oder Nahrungsmittel- oder Diätzusatz nach Anspruch **13,** umfassend 50-200 mg, vorzugsweise ungefähr 100 m2 SOD, 2,5-7,5 mg, vorzugsweise ungefähr 5 mg Lutein und 0.5-1,5 mg, vorzugsweise ungefähr 1 mg Zeaxanthin.

15. Funktionelles Nahrungsmittel, nutrazeutische Zusammensetzung oder Nahrungsmittel- oder Diätzusatz nach Anspruch **13** oder Anspruch **14,** in einer Form, die geeignet ist, um oral verabreicht zu werden.

16. Pharmazeutische Zusammensetzung, umfassend eine Verbindung nach einem der Ansprüche **1** bis **9** oder einen Satz von Teilen nach einem der Ansprüche **10** bis **12** in Verbindung mit mindestens einem pharmazeutisch akzeptablen Trägerstoff.

17. Arzneimittel, umfassend eine Zusammensetzung nach einem der Ansprüche **1** bis **9** oder einen Satz von Teilen nach einem der Ansprüche **10** bis **12.**

18. Pharmazeutische Zusammensetzung nach Anspruch **16** oder Arzneimittel nach Anspruch **17,** umfassend 100-1000 mg Superoxiddismutase, 1-25 mg Lutein und 0,5-5 mg Zeaxanthin.

19. Pharmazeutische Zusammensetzung oder Arzneimittel nach einem der Ansprüche **16** bis **18** in einer Form, die geeignet ist, um oral, topisch, intraocular oder systemisch verabreicht zu werden

20. Tierärztliches Produkt, umfassend eine Zusammensetzung nach einem der Ansprüche **1** bis **9** oder einen Satz von Teilen nach einem der Ansprüche **10** bis **12.**

## Revendications

1. Composition comprenant de la superoxide dismutase (SOD) en association avec de la lutéine et au moins un isomère de zéaxanthine.

2. Composition selon la revendication **1,** dans laquelle la SOD est d'origine naturelle, et la composition comprend un extrait végétal incluant la SOD, de préférence un extrait de *Cucumis melo* LC, de préférence la quantité d'extrait végétal incluant la superoxide dismutase varie de 10 à 2000 mg, de préférence de 50 à 1000 mg, plus préférentiellement 100-500 mg.

3. Composition selon la revendication **1** ou la revendication **2,** dans laquelle l'activité enzymatique de la SOD est de au moins 1.1 UI/mg.

4. Composition selon l'une quelconque des revendications **1** à **3,** dans laquelle la SOD est micro-encapsulée avec un biopolymère, de préférence une prolamine ou PCADK.

5. Composition selon la revendication **4,** dans laquelle la SOD micro-encapsulée est en outre revêtue d'un polymère, de préférence le chitosan ou l'acide hyaluronique.

6. Composition selon l'une quelconque des revendications **1** à **5,** dans laquelle l'au moins un isomère de zéaxanthine est choisi dans le groupe constitué par la zéaxanthine et la mésozéaxanthine.

7. Composition selon l'une quelconque des revendications **1** à **6,** dans laquelle la quantité de lutéine varie de 0.5 à 25 mg, et la quantité d'au moins un isomère de zéaxanthine varie de 0.1-5 mg.

8. Composition selon l'une quelconque des revendications **1** à **7,** dans laquelle la lutéine et l'au moins un isomère de zéaxanthine sont présents dans la composition dans un ratio lutéine : isomère de zéaxanthine d'environ 5 :1.

9. Composition selon l'une quelconque des revendications **1** à **8,** dans laquelle l'association est un mélange intime de superoxide dismutase, de lutéine et d'au moins un isomère de zéaxanthine.

10. Kit d'éléments comprenant une composition selon l'une quelconque des revendications **1** à **9,** dans lequel le kit comprend un premier élément comprenant de la superoxide dismutase, et un second élément comprenant de la lutéine et au moins un isomère de zéaxanthine.

11. Kit selon la revendication **10,** dans lequel un des premier et second élément est formulé pour être libéré de façon prolongée.

12. Kit selon la revendication **10** ou la revendication **11,** dans lequel le premier et le second éléments sont à administrer à un individu simultanément ou séquentiellement, et quand ils sont administrés séquentiellement, le premier élément comprenant la SOD est à administrer 12 heures avant ou après le second élément comprenant la lutéine et au moins un isomère de zéaxanthine.

13. Alicament, composition nutraceutique ou supplément alimentaire ou diététique comprenant une composition selon l'une quelconque des revendications **1** à **9** ou un kit d'éléments selon l'une quelconque des revendications **10** à **12.**

14. Alicament, composition nutraceutique ou supplément alimentaire ou diététique selon la revendication **13,** comprenant 50-200 mg, de préférence environ 100 mg de SOD, 2.5-7.5 mg, de préférence environ 5 mg de lutéine et 0.5-1.5 mg, de préférence environ 1 mg de zéaxanthine.

15. Alicament, composition nutraceutique ou supplément alimentaire ou diététique selon la revendication **13** ou la revendication **14,** sous une forme adaptée pour être administré oralement.

16. Composition pharmaceutique comprenant une composition selon l'une quelconque des revendications **1** à **9** ou un kit d'éléments selon l'une quelconque des revendications **10** à **12** en association avec au moins un excipient pharmaceutiquement acceptable.

17. Médicament comprenant une composition selon l'une quelconque des revendications **1** à **9** ou un kit d'éléments selon l'une quelconque des revendications **10** à **12.**

18. Composition pharmaceutique selon la revendication **16** ou médicament selon la revendication **17,** comprenant 100-1000 mg de superoxide dismutase, 1-25 mg de lutéine et 0.5-5 mg de zéaxanthine.

19. Composition pharmaceutique ou médicament selon l'une quelconque des revendications **16** à **18,** sous une forme adaptée pour être administré par voie orale, topique, intraoculaire ou systémique.

20. Produit vétérinaire, comprenant une composition selon l'une quelconque des revendications **1** à **9** ou un kit d'éléments selon l'une quelconque des revendications **10** à **12.**
